# EUROPEAN PATENT APPLICATION

(11) **EP 2 832 295 A1**
(43) Date of publication of application: **04.02.2015**
(21) Application number: 12872679.1
(22) Date of filing: 18.12.2012
(51) Int. Cl.: A61B 8/00

(54) **ULTRASONIC PROBE AND ULTRASONIC DIAGNOSTIC DEVICE COMPRISING SAME**

(30) Priority: 30.03.2012 JP 2012080793
(71) Applicant: FUJIFILM Corporation, Tokyo 106-8620 (JP)
(72) Inventor: OSAWA, Atsushi, Ashigara-kami-gun Kanagawa 258-8538 (JP)
(74) Representative: Klunker . Schmitt-Nilson . Hirsch
(86) International application number: PCT/JP2012/082754
(87) International publication number: WO 2013/145466

(57) **Abstract**

Provided is an ultrasonic probe (4) comprising a plurality of impedance matching elements (7) connected to a plurality of ultrasonic transducers (a1-a2n) and having a prescribed matching frequency in a transmission path (6) for reception signals. In order to reduce the effect of ultrasonic transmission/reception signal angles, caused by arrangement positions in an array (11) for the corresponding ultrasonic transducer, the matching frequencies for the plurality of impedance matching elements (7) have a distribution such that the matching frequencies increase the closer the corresponding arrangement position is to the center. Reception signals received by the ultrasonic transducers (a1-a2n) for the ultrasonic probe (4) pass through each impedance matching element (7) comprising a prescribed frequency distribution and arranged in each transmission path (6), and as a result: the effect of displacement of a central frequency for each reception signal is compensated, the reception signals are sequentially output to a diagnostic device main body (5), and reception data is generated; and a high image-quality ultrasonic image is generated on the basis of this reception data.

## Description

### TECHNICAL FIELD

The present invention relates to an ultrasound probe and an ultrasound diagnostic device including the ultrasound probe, and more particularly, to an ultrasound probe that is connected to a diagnostic device body and that transmits and receives ultrasonic waves.

### BACKGROUND ART

An ultrasound diagnostic device using an ultrasound image has hitherto been put into practical use in the field of medicine. In general, in such a type of ultrasound diagnostic device, an ultrasonic beam is transmitted from an array transducer of an ultrasound probe toward a subject, an ultrasonic echo from the subject is received by the array transducer, and the received signal is electrically processed in a diagnostic device body to generate an ultrasound image.

Patent Literature 1 discloses an ultrasound diagnostic device equipped with a matching unit that performs impedance matching by installing an inductor element connected in series and a capacitor element connected in parallel between an ultrasound vibrator and a transmission/reception unit connected to the ultrasound vibrator via a signal line.

Patent Literature 2 and Patent Literature 3 disclose an ultrasound diagnostic device in which a tuning frequency is variable so as to receive reflected waves reflected from different depths in a best state.

According to the ultrasound diagnostic device, it is possible to obtain an optimal reception signal depending on the focal depth by changing the tuning frequency through impedance matching.

### CITATION LIST

### PATENT LITERATURE

Patent Literature 1: JP 3702259 B
Patent Literature 2: JP 06-225881 A
Patent Literature 3: JP 63-221241 A

### SUMMARY OF INVENTION

### TECHNICAL PROBLEMS

In the conventional ultrasound diagnostic device, impedance matching is carried out depending on the site or depth to be diagnosed as described above, but with respect to the transducers, only characteristics of ultrasonic echo when ultrasonic waves are radiated toward the front direction are considered, and degradation in image quality of an ultrasound image due to a difference in arrangement position between the individual transducers which are arranged in an array , that is, a difference in an incidence angle of the ultrasonic echo on the individual ultrasound transducers is not considered at all.

An object of the invention is to provide an ultrasound probe that can compensate for degradation in image quality of an ultrasound image due to a difference in the incidence angle of the ultrasonic echo on a plurality of ultrasound transducers arranged in an array, and to provide an ultrasound diagnostic device having the ultrasound probe.

### SOLUTION TO PROBLEMS

In order to achieve the above-mentioned object, according to an aspect of the invention, there is provided an ultrasound probe connected to a diagnostic device body, and for generating reception signals by transmitting and receiving ultrasonic waves through a plurality of ultrasound transducers arranged in an array and transmitting the generated reception signals to the diagnostic device body, wherein a plurality of impedance matching elements connected to the plurality of ultrasound transducers and each having a respective preset matching frequency are disposed in transmission paths of the reception signals, and wherein the plurality of impedance matching elements have a distribution in which a matching frequency of an impedance matching element becomes higher as an arrangement position of an ultrasound transducer corresponding to the impedance matching element in the array of the plurality of ultrasound transducers becomes closer to a center of the array, in order to reduce an influence of transmission/reception angles of ultrasonic waves due to the arrangement position of the corresponding ultrasound transducer in the array.

Each of the impedance matching elements may be constituted by connecting at least one inductor or capacitor in series or in parallel.

The ultrasound probe preferably comprises a probe body that includes the plurality of ultrasound transducers, a cable that connects the probe body to the diagnostic device body, and a connector that connects the cable to the diagnostic device body. Most preferably, the plurality of impedance matching elements is disposed in the connector. The plurality of impedance matching elements is preferably disposed in the probe body. The plurality of impedance matching elements may be disposed in the cable.

According to another aspect of the invention, there is provided an ultrasound diagnostic device including the above-mentioned ultrasound probe and a diagnostic device body to which the ultrasound probe is connected.

### ADVANTAGEOUS EFFECTS OF INVENTION

According to the invention, since the influence of a difference in central frequency between individual reception signals due to arrangement positions of the plurality of ultrasound transducers arranged in an array can be compensated for, it is possible to acquire an ultrasound image with high image quality.

### BRIEF DESCRIPTION OF DRAWINGS

FIG. 1 is a block diagram illustrating an internal configuration of an ultrasound probe according to Embodiment 1 of the invention.
FIG. 2 is a diagram schematically illustrating a positional relationship between a reflection point in a subject and individual ultrasound transducers of the ultrasound probe.
FIG. 3 is a graph illustrating a relationship between installation positions of individual ultrasound transducers of the ultrasound probe according to Embodiment 1 and matching frequencies of corresponding impedance matching elements.
FIGS. 4A to 4F are diagrams illustrating an example of an impedance matching element installed in a transmission path of the ultrasound probe according to Embodiment 1.
FIG. 5 is a block diagram illustrating an internal configuration of a diagnostic device body connected to the ultrasound probe.
FIG. 6 is a block diagram illustrating an internal configuration of an ultrasound probe according to Embodiment 2 of the invention.
FIG. 7 is a block diagram illustrating an internal configuration of an ultrasound probe according to a modification example of Embodiment 2 of the invention.
FIG. 8 is a block diagram illustrating an internal configuration of a diagnostic device body connected to the ultrasound probe illustrated in FIG. 7.
FIG. 9 is a block diagram illustrating an internal configuration of an ultrasound probe according to Embodiment 3 of the invention.

### DESCRIPTION OF EMBODIMENTS

Hereinafter, embodiments of the invention will be described with reference to the accompanying drawings.

### Embodiment 1

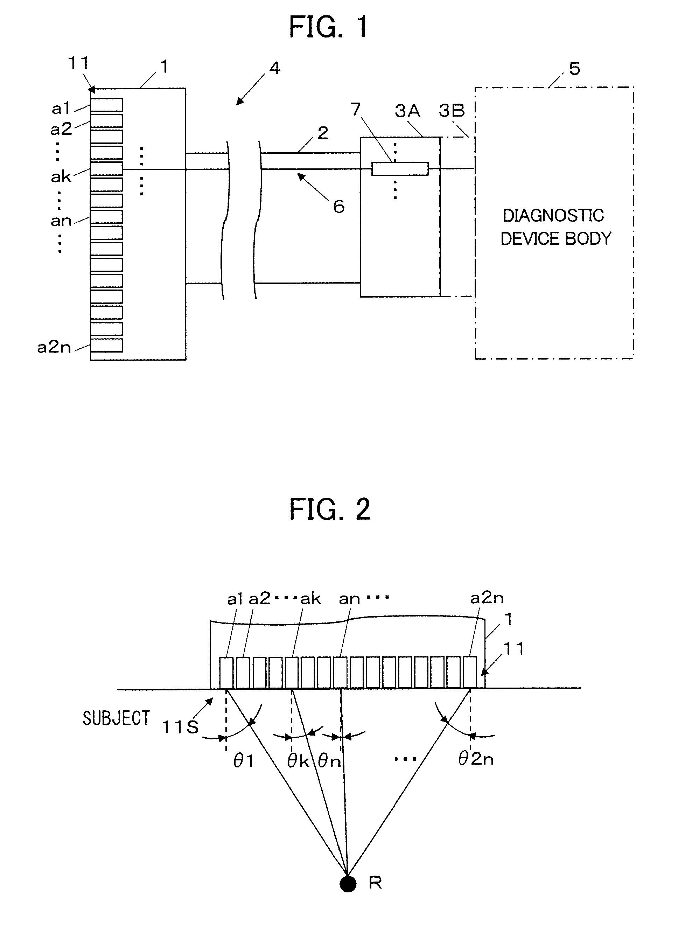

FIG. 1 illustrates a configuration of an ultrasound probe 4 according to Embodiment 1 of the invention. The ultrasound probe 4 is connected to a diagnostic device body 5 via connectors 3A and 3B to constitute an ultrasound diagnostic device.

The ultrasound probe 4 includes, for example, a probe body 1 that has an array transducer 11 composed of plural ultrasound transducers a1 to a2n which are arranged in a one-dimensional array, a cable 2 that is connected to the probe body 1 and connects the probe body 1 to the diagnostic device body 5, and a connector 3A that connects the cable 2 to the connector 3B of the diagnostic device body 5. The cable 2 is fixedly connected to the probe body 1 but is connected to the diagnostic device body 5 via the connectors 3A and 3B so as to be detachable.

Each of the plural ultrasound transducers a1 to a2n is independently connected to the diagnostic device body 5 via each transmission path 6, and an impedance matching element 7 is installed in the connector 3A in the middle of each of the transmission paths 6.

Each of the plural ultrasound transducers a1 to a2n transmits ultrasonic wave in response to a driving signal supplied from a transmission circuit 12 via the individual transmission path 6 and receives an ultrasonic echo from a subject and outputs a reception signal. Each ultrasound transducer ak (where k=1 to 2n) is constituted by a vibrator in which electrodes are formed on both ends of a piezoelectric substance formed of, for example, a piezoelectric ceramic represented by PZT (lead zirconate titanate), a polymer piezoelectric element represented by PVDF (polyvinylidene fluoride), a piezoelectric single crystal represented by PMN-PT (lead magnesium niobate-lead titanate solid solution), or the like.

When a pulsed or continuous-wave voltage is applied across the electrodes of each vibrator, the piezoelectric substance expands and contracts, whereby pulsed or continuous-wave ultrasonic waves are generated from the respective vibrators, and an ultrasonic beam is formed by synthesis of the ultrasonic waves generated. When receiving propagating ultrasonic waves, the respective vibrators expand and contract and generate electrical signals. The electrical signals are output as reception signals of the ultrasonic waves.

FIG. 2 illustrates a positional relationship between the array transducer 11 of the probe body 1 and a predetermined reflection point R in the subject. The plural ultrasound transducers a1 to a2n of the array transducer 11 are one-dimensionally arranged and have acoustic radiation surfaces 11S parallel to each other. The one reflection point R is located to face the acoustic radiation surfaces 11S of the plural ultrasound transducers a1 to a2n. Accordingly, the angles formed by the reflection point R and the acoustic radiation surfaces 11S of the respective ultrasound transducers ak are different depending on the ultrasound transducers ak. Here, the angle θk (where k=1 to 2n) formed by the acoustic radiation surface 11S of the ultrasound transducer ak is expressed by an angle formed by a straight line connecting the acoustic radiation surface 11S of the ultrasound transducer ak to the reflection point R and the normal line of the acoustic radiation surface 11S. The angle θk formed by the acoustic radiation surface 11S of the ultrasound transducer ak is also an incidence angle of the ultrasonic echo on the ultrasound transducer ak from the reflection point R. When the reflection point R is located on the center line of the array transducer 11, the ultrasound transducers located at symmetric positions with respect to the center line have the same incidence angles of ultrasonic echo. That is, the ultrasound transducers a1 and a2n, the ultrasound transducers a2 and a (2n-1), the ultrasound transducers a3 and a (2n-2), ..., the ultrasound transducers an to a (n+1) have the same incidence angles of ultrasonic echo.

As described above, since the incidence angle θk of the ultrasonic echo returned from the reflection point R differs depending on the ultrasound transducers ak, the reception signals obtained from the ultrasound transducers ak have different central frequencies depending on the incidence angle θk of the ultrasonic echo. In general, the larger the incidence angle θk of the ultrasonic echo becomes, the lower the central frequency of the reception signal obtained by the ultrasound transducer ak becomes.

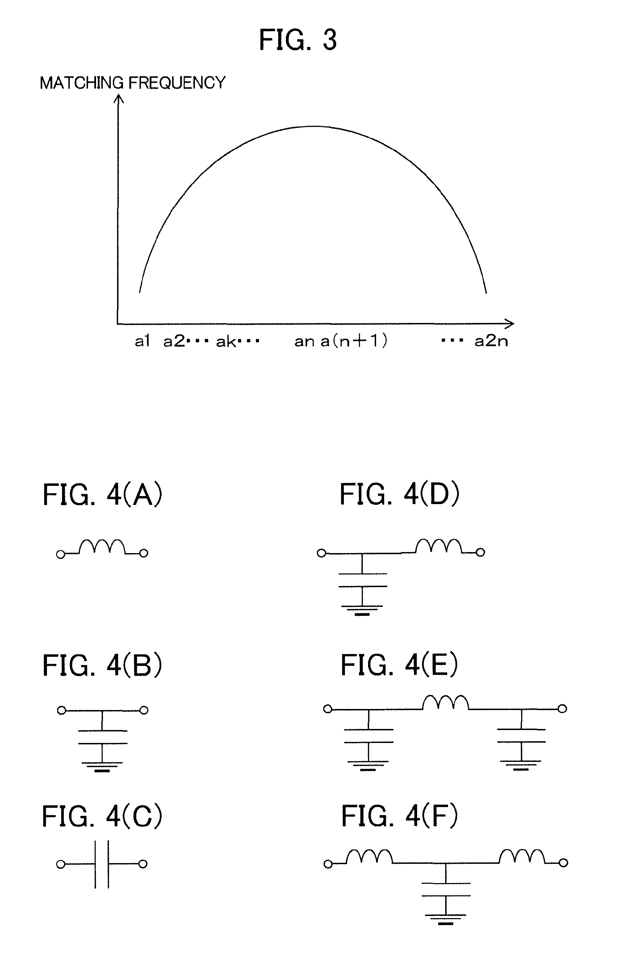

If the reception signals having different central frequencies are subjected to phase matching as they are to generate a sound-ray signal, degradation in image quality of an ultrasound image is caused. Accordingly, in the ultrasound probe 4, in order to compensate for an influence of a difference in central frequency between the individual reception signals from the ultrasound transducers ak, the plural impedance matching elements 7, which are installed in the respective transmission paths 6 in the connector 3A for the plural ultrasound transducers a1 to a2n arranged in an array, are adjusted so as to have a distribution of matching frequencies illustrated in FIG. 3. As described above, since the ultrasound transducers located at symmetric positions with respect to the center line of the array transducer 11 passing through the reflection point R have the same incidence angles of ultrasonic echo, the distribution of matching frequencies illustrated in FIG. 3 also has a distribution which is symmetric with respect to the center.

In the ultrasound probe 4 according to Embodiment 1, since the plural impedance matching elements 7 installed in the transmission paths 6 of the individual reception signals have a frequency distribution corresponding to the arrangement positions of the ultrasound transducers a1 to a2n, and thus, the influence of the difference in central frequency between the individual reception signals is compensated for by the individual impedance matching elements 7. Therefore, when the ultrasound probe 4 is connected to the diagnostic device body 5, it is possible to generate an ultrasound image with high image quality.

Each impedance matching element 7 is to adjust electric impedance. For example, the impedance matching element 7 may be an inductor which is connected in series to each transmission path 6 as illustrated in FIG. 4A, may be a capacitor which is connected in parallel thereto as illustrated in FIG. 4B, may be a capacitor which is connected in series thereto as illustrated in FIG. 4C, or may be a capacitor and an inductor connected in parallel which are connected in series thereto as illustrated in FIG. 4D.

In addition, as illustrated in FIG. 4E, π-type matching which is used when the electric impedance on the vibrator side is high mainly for the purpose of improvement of reception sensitivity, or as illustrated in FIG. 4F, T-type matching which is used when the electric impedance on the vibrator side is low mainly for the purpose of improvement of reception sensitivity may be used.

By causing the reception signals from the ultrasound transducers ak to pass through the corresponding impedance matching elements 7, the influence of the difference in central frequency between the reception signals is compensated for.

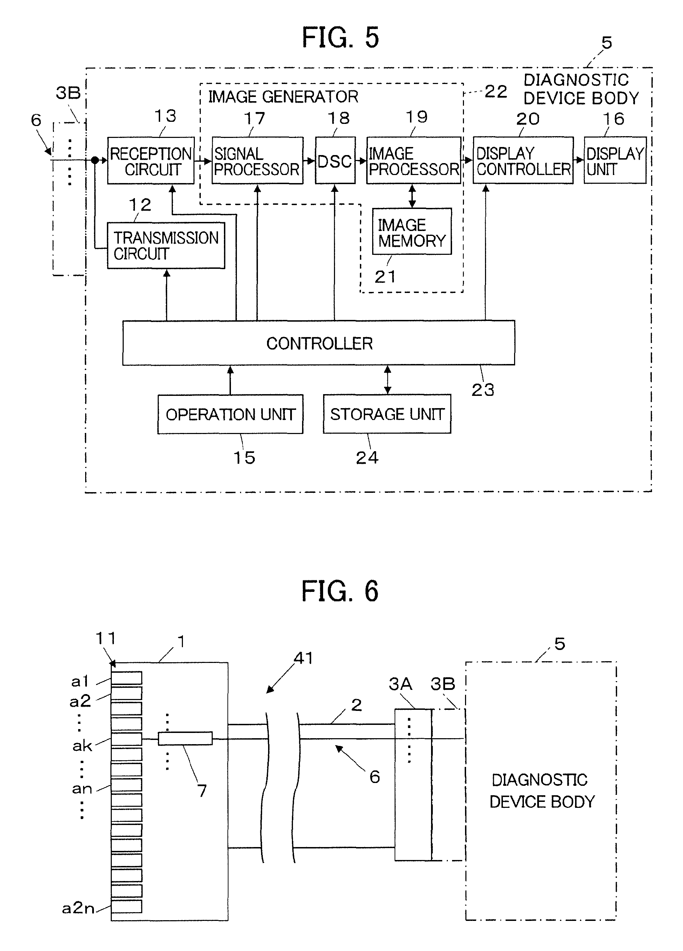

Next, the internal configuration of the diagnostic device body 5 is illustrated in FIG. 5.

The diagnostic device body 5 includes a transmission circuit 12 and a reception circuit 13 connected to the plural ultrasound transducers a1 to a2n of the probe body 1 via the respective transmission paths 6.

The diagnostic device body 5 includes a signal processor 17 connected to the reception circuit 13, and a DSC (digital scan converter) 18, an image processor 19, a display controller 20, and a display unit 16 are sequentially connected to the signal processor 17. An image memory 21 is connected to the image processor 19, and an image generator 22 is constituted by the signal processor 17, the DSC 18, the image processor 19, and the image memory 21.

Further, a controller 23 is connected to the transmission circuit 12, the reception circuit 13, the signal processor 17, the DSC 18, and the display controller 20, and an operation unit 15 and a storage unit 24 are respectively connected to the controller 23.

The transmission circuit 12 illustrated in FIG. 5 includes, for example, plural pulsers, adjusts a delay amount of each of driving signals on the basis of a transmission delay pattern selected depending on a control signal from the controller 23 such that the ultrasonic waves transmitted from the plural ultrasound transducers a1 to a2n of the array transducer 11 illustrated in FIG. 1 form an ultrasonic beam, and supplies the adjusted driving signals to the plural ultrasound transducers a1 to a2n.

The reception circuit illustrated in FIG. 5 amplifies and A/D converts the reception signals transmitted from the ultrasound transducers ak, and then performs a reception focusing processing by giving a delay to each of the reception signals and adding the reception signals according to a sound velocity or a sound velocity distribution set based on a reception delay pattern selected depending on the control signal from the controller 23. Reception data (sound-ray signal) in which the focus of ultrasonic echo is narrowed down is generated by this reception focusing processing.

The signal processor 17 of the diagnostic device body 5 performs correction of attenuation due to distance on the reception data generated by the reception circuit 13 depending on the depth of the reflection position of ultrasonic waves, and then performs an envelope detection processing to generate a B-mode image signal which is tomographic image information relating to the tissues in the subject.

The DSC 18 converts (raster-converts) the B-mode image signal generated by the signal processor 17 into an image signal based on a scanning method of a normal television signal.

The image processor 19 performs various necessary image processing such as gradation processing on the B-mode image signal input from the DSC 18, and then outputs the B-mode image signal to the display controller 20 or stores the B-mode image signal in the image memory 21.

The display controller 20 causes the display unit 16 to display an ultrasound diagnostic image on the basis of the B-mode image signal subjected to the image processing by the image processor 19.

The display unit 16 includes, for example, a display device such as an LCD and displays an ultrasound diagnostic image under the control of the display controller 24. At the time of examination, a cursor or a caliper for examination is displayed to overlap with the ultrasound diagnostic image if necessary.

The operation unit 15 includes various operation buttons for allowing an operator to perform an input operation. The storage unit 24 stores an operation program or an examination program including a series of examination items, and a recording medium such as a hard disk, a flexible disk, an MO, an MT, a RAM, a CD-ROM, a DVD-ROM, an SD card, a CF card, and a USB memory or a server can be used as the storage unit 24.

The controller 23 controls the respective constituents of the diagnostic device body 5 on the basis of various command signals input from the operation unit 15 by the operator.

Operation programs for operating the signal processor 17, the DSC 18, the image processor 19, and the display controller 20 may be constituted by digital circuits.

Next, the operation of the ultrasound diagnostic device in which the ultrasound probe 4 according to Embodiment 1 is connected to the diagnostic device body 5 will be described below.

By connecting the ultrasound probe 4 to the diagnostic device body 5 via the connectors 3A and 3B and turning on a power switch of the diagnostic device body 5, power is supplied to the respective constituents of the diagnostic device body 5 and the ultrasound probe 4 and the ultrasound diagnostic device is started.

An ultrasonic beam is sequentially transmitted from the plural ultrasound transducers a1 to a2n of the array transducer 11 in response to the driving signal from the transmission circuit 12 of the diagnostic device body 5, the influence of the difference in central frequency between the individual reception signals received by the plural ultrasound transducers a1 to a2n is compensated by causing the reception signals to pass through the individual impedance matching elements 7 which are installed in the individual transmission paths 6 and have a predetermined frequency distribution, and the reception signals are sequentially output to the reception circuit 13 of the diagnostic device body 5 via the transmission paths 6 to generate reception data. On the basis of the reception data, an image signal is generated by the image generator 22 of the diagnostic device body 5, and on the basis of the image signal, an ultrasound image is displayed on the display unit 16 by the display controller 20.

As described above, since the influence of the difference in central frequency between the reception signals is compensated by the impedance matching elements 7 corresponding to the arrangement positions of the ultrasound transducers ak installed in the connector 3A, it is possible to obtain an ultrasound image with high image quality.

In Embodiment 1, the influence of the difference in central frequency between the plural reception signals is compensated by the corresponding impedance matching elements 7 having a predetermined matching frequency distribution, and since the impedance matching elements 7 are installed in the connector 3A of the ultrasound probe 4, it is possible to achieve a decrease in size of the probe body 1, and it is thus easy to operate the ultrasound probe 4.

### Embodiment 2

FIG. 6 illustrates the configuration of an ultrasound probe 41 according to Embodiment 2. The ultrasound probe 41 is different from the ultrasound probe 4 according to Embodiment 1 illustrated in FIG. 1, in that the impedance matching elements 7 are installed in the probe body 1 instead of the connector 3A, and the other configurations are the same as those of the ultrasound probe 4 according to Embodiment 1.

Even when the impedance matching elements 7 connected to the plural ultrasound transducers a1 to a2n of the array transducer 11 are disposed in the probe body 1 in this way, the same operations as in Embodiment 1 can be carried out to compensate for the influence of the difference in central frequency between the individual ultrasound transducers a1 to a2n, thereby obtaining an ultrasound image with high image quality.

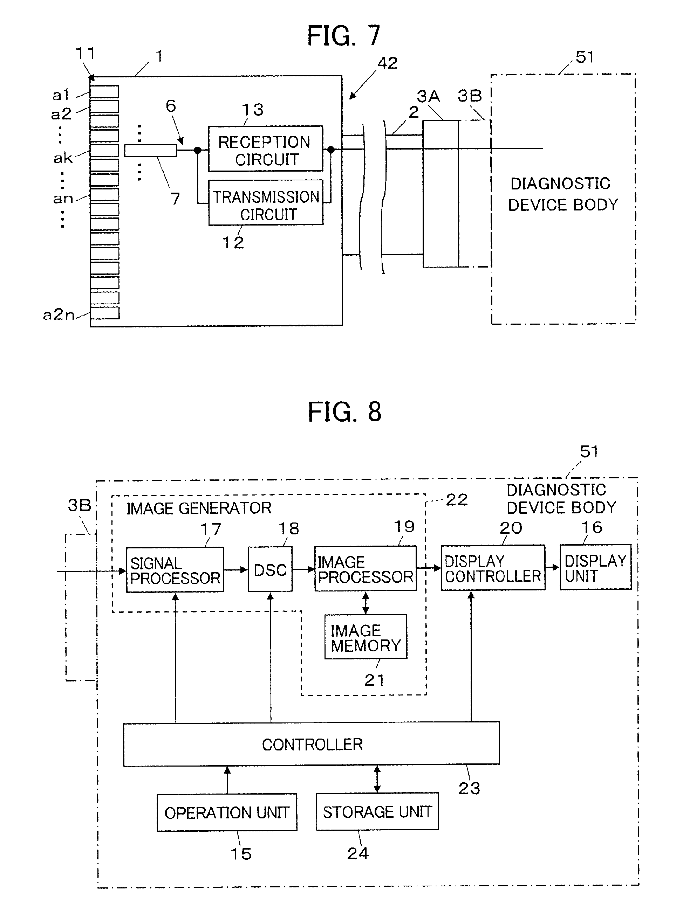

Further, as a modification example of Embodiment 2, an ultrasound probe 42 may be constituted such that the transmission circuit 12 and the reception circuit 13, which are disposed in the diagnostic device body 5 in Embodiment 1, are also disposed in the probe body 1, as illustrated in FIG. 7.

In this case, the ultrasound probe 42 is connected to a diagnostic device body 51 illustrated in FIG. 8 for use. The diagnostic device body 51 is obtained by removing the transmission circuit 12 and the reception circuit 13 from the diagnostic device body 5 illustrated in FIG. 5.

In the ultrasound probe 42, the reception signals obtained by the plural ultrasound transducers a1 to a2n of the array transducer 11 are respectively input to the reception circuit 13 via the impedance matching elements 7 and signals digitalized by the reception circuit 13 are transmitted to the diagnostic device body 51. That is, a digital ultrasound probe 42 is constituted.

### Embodiment 3

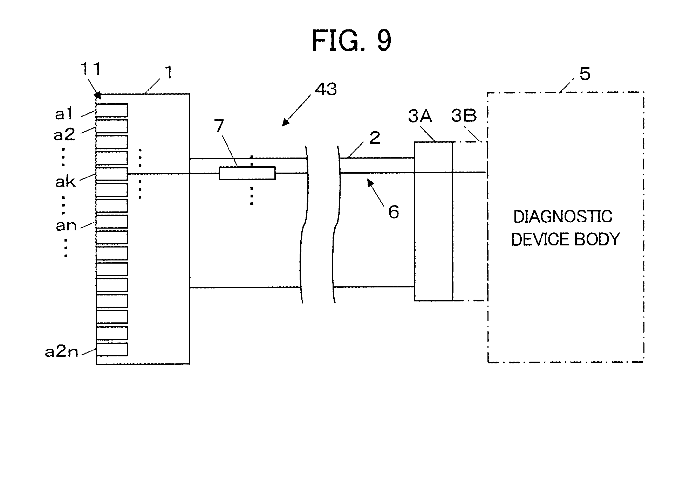

FIG. 9 illustrates the configuration of an ultrasound probe 43 according to Embodiment 3. The ultrasound probe 43 is different from the ultrasound probe 4 according to Embodiment 1 illustrated in FIG. 1, in that the impedance matching elements 7 are installed in the cable 2 instead of the connector 3A, and the other configurations are the same as those of the ultrasound probe 4 according to Embodiment 1.

Even when the impedance matching elements 7 connected to the plural ultrasound transducers a1 to a2n of the array transducer 11 are disposed in the cable 2 in this way, the same operations as in Embodiment 1 can be carried out to compensate for the influence of the difference in central frequency between the individual ultrasound transducers a1 to a2n, thereby obtaining an ultrasound image with high image quality.

In Embodiments 1 and 2, the ultrasound probe 4 or 41 and the diagnostic device body 5 are connected to each other via the cable 2, and the ultrasound probe 42 and the diagnostic device body 51 are connected to each other via the cable 2, but the invention is not limited to this configuration. The probe body 1 may be connected to the diagnostic device body 5 or 51 in a wireless manner.

So far, the ultrasound probe and the ultrasound diagnostic device including the ultrasound probe according to the invention have been described in detail, but the invention is not limited to the above-described embodiments and may be improved or modified in various forms within the scope that does not depart from the gist of the invention.

### DESCRIPTION OF SYMBOLS

- 1:: PROBE BODY
- 2:: CABLE
- 3A, 3B:: CONNECTOR
- 4:: ULTRASOUND PROBE
- 5:: DIAGNOSTIC DEVICE BODY
- 6:: TRANSMISSION PATH
- 7:: IMPEDANCE MATCHING ELEMENT
- 8:: DISPLAY UNIT
- 9:: TOUCH PANEL
- 11:: ARRAY TRANSDUCER
- 11S:: ACOUSTIC RADIATION SURFACE
- 12:: TRANSMISSION CIRCUIT
- 13:: RECEPTION CIRCUIT
- 15:: OPERATION UNIT
- 16:: DISPLAY UNIT
- 17:: SIGNAL PROCESSOR
- 18:: DSC (Digital Scan Converter)
- 19:: IMAGE PROCESSOR
- 20:: DISPLAY CONTROLLER
- 21:: IMAGE MEMORY
- 22:: IMAGE GENERATOR
- 23:: CONTROLLER
- 24:: STORAGE UNIT
- a1∼a2n:: ULTRASOUND TRANSDUCER

## Claims

1. An ultrasound probe connected to a diagnostic device body, and for generating reception signals by transmitting and receiving ultrasonic waves through a plurality of ultrasound transducers arranged in an array and transmitting the generated reception signals to the diagnostic device body,
wherein a plurality of impedance matching elements connected to the plurality of ultrasound transducers and each having a respective preset matching frequency are disposed in transmission paths of the reception signals, and
wherein the plurality of impedance matching elements have a distribution in which a matching frequency of an impedance matching element becomes higher as an arrangement position of an ultrasound transducer corresponding to the impedance matching element in the array of the plurality of ultrasound transducers becomes closer to a center of the array, in order to reduce an influence of transmission/reception angles of ultrasonic waves due to the arrangement position of the corresponding ultrasound transducer in the array.

2. The ultrasound probe according to claim 1,
wherein each of the impedance matching elements is constituted by connecting at least one inductor or capacitor in series or in parallel.

3. The ultrasound probe according to claim 1 or 2,
wherein the ultrasound probe comprises a probe body that includes the plurality of ultrasound transducers, a cable that connects the probe body to the diagnostic device body, and a connector that connects the cable to the diagnostic device body, and
wherein the plurality of impedance matching elements are disposed in the connector.

4. The ultrasound probe according to claim 1 or 2,
wherein the ultrasound probe comprises a probe body that includes the plurality of ultrasound transducers, a cable that connects the probe body to the diagnostic device body, and a connector that connects the cable to the diagnostic device body, and
wherein the plurality of impedance matching elements are disposed in the probe body.

5. The ultrasound probe according to claim 1 or 2,
wherein the ultrasound probe comprises a probe body that includes the plurality of ultrasound transducers, a cable that connects the probe body to the diagnostic device body, and a connector that connects the cable to the diagnostic device body, and
wherein the plurality of impedance matching elements are disposed in the cable.

6. An ultrasound diagnostic device comprising:
the ultrasound probe according to any one of claims 1 to 5; and
a diagnostic device body to which the ultrasound probe is connected.
